# EUROPEAN PATENT APPLICATION

(11) **EP 4 599 690 A1**
(43) Date of publication of application: **13.08.2025**
(21) Application number: 24305229.7
(22) Date of filing: 12.02.2024
(51) Int. Cl.: A23K 20/163, A23L 29/30, A23L 33/125, A23L 33/20, A23L 33/21, A23L 33/26, C07H 3/06, C08B 37/00

(54) **RESISTANT & SLOW DIGESTIBLE SACCHARIDE OLIGOMERS**

(71) Applicant: ROQUETTE FRERES, 62136 Lestrem (FR)
(72) Inventor: Gras, Ludovine, 62136 Lestrem (FR); Carre, Yoann, 62400 Bethune (FR); Boit, Baptiste, 59253 La Gorgue (FR)
(74) Representative: Plasseraud IP

(57) **Abstract**

The invention relates to saccharide oligomers comprising: - a total amount of DP1 - DP2 ranging from 5 and 10 %, preferably from 4 to 8 %, and a total amount of DP 3 to DP 5 ranging from 9 and 19%, preferably from 10 to 15 %; - a ratio of 1,6-glycosidic/1,4-glycosidic linkages ranging from 0.5 to about 2 preferably from 0.6 to 1.8, and a ratio of 1,4-glycosidic/1,2-glycosidic linkages ranging from 1,5 to 6, preferably from 1.9 to 5. It also relates to a process for preparing the saccharide oligomers and to a food composition comprising the saccharide oligomers.

## Description

The present invention relates, according to a first embodiment, to saccharide oligomers that are digestion resistant or slowly digestible, comprising a total amount of DP1 - DP2 ranging from 4 and 10%, preferably from 4 to 8 %, a total amount of DP 3 to DP 5 ranging from 9 and 19%, preferably from 10 to 15 %, a ratio of 1,6-glycosidic/1,4-glycosidic ranging from 0.5 to about 2 preferably from 0.6 to 1.8, and a ratio of 1,4-glycosidic/1,2-glycosidic linkages ranging from 1,5 to 6, preferably from 1.9 to 5.

According to a second embodiment, the saccharide oligomers especially have a ratio of (DP3-5)/DP6+ less than 0.23 (DP6+ meaning DP greater than or equal to 6).

According to a third embodiment, the saccharide oligomers especially have a Mw ranging from 2000 to 3000 daltons and an Mn ranging from 900 to 1500 daltons.

According to a fourth embodiment, the saccharide oligomers especially have a fiber content of between 60 and 90 %, more preferably 70 to 85 %.

Particularly advantageously, fine-tuning the content of α-1,2-glycosidic, α-1,4-glycosidic and α-1,6-glycosidic bonds leads to unique products in accordance with the present invention, demonstrating an ideal compromise between relatively high fiber-richness and very low glucose bioavailability with regard to the organism.

Thus, as yet unidentified mixed products are available which afford all the benefits of fiber-based foods, that is particularly advantageous for use in healthy balanced diets.

The invention also relates to an original method which is very simple to implement for manufacturing these saccharide oligomers, in which a saccharified corn starch syrup with a specific DP1 and DP2 content (a DP1 content of between 45 and 60 % by dry weight and a DP2 content of between 30 and 40 %) undergoes an acid and heat treatment at high temperature (between 100° C and 150° C) under absolute pressure between 200 to 1000 mbar, followed by a fiber enrichment method chosen in the group consisting of chromatography, membrane filtration with or without preliminary enzymatic treatment.

The choice of starting raw materials, and especially of a blend of the DP1 and DP2-rich aqueous carbohydrate solution, but also the final purification, are some of the means by which the content of α-1,2-glycosidic, α-1,4-glycosidic and α-1,6-glycosidic- bonds can be regulated to within the range mentioned above.

A final subject of the present invention consists of the use of the saccharide oligomers according to the invention in human food and animal feed.

### TECHNICAL PROBLEM AND PRIOR ART

For several years, there has been some interest among the general public for new, fiber-based diets. The term "dietary fiber" is intended to mean plant parts which are not hydrolyzed by enzymes during digestion. They are residual substances which come from the cell wall or cytoplasm of plants, consisting of complex mixtures of carbohydrates which have been identified as non-starch polysaccharides.

Among dietary fiber, a distinction is made between insoluble fibers and water-soluble fibers. Oats, barley, fruits, fresh vegetables and pulses (beans, lentils, chickpeas) constitute good sources of soluble fibers, whereas whole grain cereals and whole grain bread are rich in insoluble fibers. Insoluble fibers, such as cellulose, resistant starches, corn fibers (spent grain) or soya fibers, have an essentially mechanical role in the gastrointestinal tract. They are only very slightly fermented by colonic flora, and contribute to reducing intestinal transit time by a ballast effect. Insoluble fibers thus contribute to preventing constipation by increasing the weight of the stools and by reducing the duration of intestinal transit.

Soluble fibers, such as pectin and inulin, which cannot be digested by the intestinal enzymes in humans or animals, are fermented by the colonic flora. This fermentation releases short-chain fatty acids in the colon, the effect of which is to reduce the pH thereof and, consequently, to limit the development of pathogenic bacteria and to stimulate the development of beneficial bacteria.

Alongside these compounds which are predominantly extracted from plants, there are molecules derived from starch or from the products of the partial or total hydrolysis thereof.

Polydextrose, for example, is synthesized by random polymerization of glucose in the presence of sorbitol and a suitable acid catalyst (such as citric acid) at high temperature. Said polydextrose is widely used in food as a filling agent and as a low-calorie ingredient.

More generally, glucose polymers are conventionally manufactured industrially by hydrolysis of natural or hybrid starches and derivatives thereof.

These starch hydrolyzates (dextrins, pyrodextrins, etc.) are thus produced by acid or enzymatic hydrolysis of cereal starch or tuber starch. They actually consist of a mixture of glucose and glucose polymers of highly varied molecular weights. Said hydrolyzates have a broad distribution of saccharides containing both linear structures (α-1,4-glycosidic bonds) and branched structures (α-1,6-glycosidic bonds).

By way of examples, patent applications EP 0 368 451 and U.S. Pat. No. 5,264,568 describe a method for preparing pyrodextrins, the dietary fiber characteristics of which are strengthened by the action of an α-amylase or several successive α-amylases on a dextrin or pyrodextrin, in solution at high temperature.

In patent application EP 0 530 111, indigestible dextrins obtained by extrusion of an acidified dehydrated corn starch under specific conditions are described. This treatment may be supplemented by the action of a heat-resistant α-amylase.

The applicant company itself also described, in its patent application EP 1 006 128, branched maltodextrins having between 22% and 35% 1,6-glycosidic bonds (both α and β type), a content of reducing sugars of less than 20%, a polydispersity index of less than 5 and a number-average molecular weight Mn at most equal to 4500 g/mol. These branched maltodextrins are most of all indigestible in character and consequently this reduces their calorific value by preventing their assimilation in the small intestine; they are therefore essentially a source of indigestible fibers.

The applicant company also described and protected, in its patent application WO 2013/128121, hyperbranched maltodextrins of low molecular weight, i.e. having a dextrose equivalent (DE) of between 8 and 15 and a molecular weight Mw of between 1700 and 3000 Daltons, characterized by a content of 1,6-glycosidic bonds (both α and β type) of between 30% and 45%, a content of soluble indigestible fibers of between 75% and 100% (according to AOAC method no. 2001-03) and noteworthy hypoglycemic properties which are reflected both in vitro and in situ by a limiting effect with respect to the standard maltodextrin digestion.

Patent application WO 2014/158777 is also known, which describes a carbohydrate which can be used as food ingredient (especially as a calorie-reducing agent) containing both linear and branched oligomers, the sugar content of which is between 5% and 25% by weight of the dry weight thereof, with a fiber content of between 10% and 70% of the dry weight thereof. Another of its features is having a content of polysaccharides with high molecular weights, such that the viscosity thereof is less than 16000 cPs at 100° F. and a solids content of 75%.

Patent applications U.S. Pat. No. 7,608,436 and WO 2008/085529 are also known, which describe a poorly digestible food product based on oligosaccharides. Said oligosaccharides have here a content of branched oligomers which is greater than the content of linear oligomers, and a concentration of non-linear oligomers with a degree of polymerization of greater than or equal to 3 which is greater than 20% by dry weight.

Patent application WO 2014/145276 is known, which describes compositions based on carbohydrates having a low calorific value. Various families of carbohydrates are described and claimed therein, especially through their content of molecules with a degree of polymerization of 1 and 2, of 1,6-bonds, of total 1,4- and 1,6- bonds, through the ratio of the content of 1,4- to 1,6- bonds and finally through their molecular weight.

Moreover, the products sold under the names PROMITOR^{®} (Tate & Lyle), FIBERSOL^{®} (Matsutani), LITESSE^{®} (Dupont Danisco) and NUTRIOSE^{®} (Roquette) are known, which are all more or less fiber-rich products based on polysaccharides.

It arises from the above text that there have been continual innovations at least over the last years in the field of fiber-rich products liable to have advantages for humans in the context of new, healthier and more balanced diets. It may especially be most interesting to have a product which is both fiber-rich and liable to release only a very small amount of glucose when it is digested by the organism.

### SUMMARY OF THE INVENTION

To this end, the applicant company has pursued much laborious study and research which has enabled it to develop products which meet these requirements.

According to a first embodiment, the saccharide oligomers that are digestion resistant or slowly digestible, comprise :
- a total amount of DP1 - DP2 ranging from 4 and 10 %, preferably from 4 to 8 %, and a total amount of DP 3 to DP 5 ranging from 9 and 19 %, preferably from 10 to 15 %,
- a ratio of 1,6-glycosidic/1,4-glycosidic ranging from 0.5 to about 2 preferably from 0.6 to 1.8, and a ratio of 1,4-glycosidic/1,2-glycosidic linkages ranging from 1,5 to 6, preferably from 1.9 to 5.

The term "saccharide oligomers" refers here to saccharides having a degree of polymerization DP of between 2 and 30.

According to a second embodiment, the saccharide oligomers especially have a ratio of (DP3-5)/DP6+ less than 0.23.

According to a third embodiment, the saccharide oligomers especially have a Mw ranging from 2000 to 3000 daltons and an Mn ranging from 900 to 1500 daltons.

According to a fourth embodiment, the saccharide oligomers especially have a fiber content of between 60 and 90 %, more preferably 70 to 85 %.

To access the proportions of total 1,4-, 1,6-, 1,2- and 1,3-glycosidic bonds, the "Hakomori" method is employed, which is well known to those skilled in the art. This method, described for the first time over 50 years ago (Hakomori, S., 1964, J. Biol. Chem., 55, 205) is nowadays widely used within the scientific community (see especially patent application EP 1 006 128, already cited in the present application).

Compared to the products of the prior art known to the applicant, the saccharide oligomers of the present application have quite specific contents of ratios of 1,6-glycosidic/1,4-glycosidic linkages and of 1,4-glycosidic/1,2-glycosidic linkages, for a content to DP 3 to DP5 which have hitherto never been described or achieved.

Moreover, by means of a particular process, the applicant has been able to synthesize the saccharide oligomers which are the subject of the present invention.

This process of preparing saccharide oligomers of the invention comprises the steps consisting of:
a) providing a saccharified corn starch syrup with a DP1 content of between 45 and 60 % by dry weight, a DP2 content of between 30 and 40 %,
b) optionally increasing the solids content of the aqueous solution resulting from step a) up to at least 70% by weight of the total weight thereof,
c) carrying out a heat treatment on the aqueous solution resulting from step b) at a temperature of between 100° C and 150° C with phosphoric acid in an acid ratio between 0.045 and 0.060 % by dry weight, under absolute pressure between 200 to 1000 mbar,
d) optionally decolorization and demineralization of the solution of step c),
e) carrying a fiber enrichment method chosen in the group consisting of chromatography, membrane filtration with or without preliminary enzymatic treatment,
f) optionally concentration and drying of the resulted solution.

Finally, it should be noted that throughout the present application, the contents of glycosidic bonds, the molecular weight and the fiber content are determined according to strict protocols which are the subject of a very detailed description in the experimental section relating to said application.

### DETAILED DESCRIPTION OF THE INVENTION

A first subject of the present invention consists of saccharide oligomers that are digestion resistant or slowly digestible.

According to a first embodiment, the saccharide oligomers that are digestion resistant or slowly digestible, comprise :
- a total amount of DP1 - DP2 ranging from 4 and 10 %, preferably from 4 to 8 %, and a total amount of DP 3 to DP 5 ranging from 9 and 19 %, preferably from 10 to 15 %,
- a ratio of 1,6-glycosidic/1,4-glycosidic ranging from 0.5 to about 2 preferably from 0.6 to 1.8, and a ratio of 1,4-glycosidic/1,2-glycosidic linkages ranging from 1,5 to 6, preferably from 1.9 to 5.

The term "saccharide oligomers" refers here to saccharides having a degree of polymerization DP of from at least 3 to at most 20.

The term "DPn" refers to the degree of polymerization, where "n" is the number of monomeric units (e.g., glucose or dextrose units) in the saccharide. For example, DP1 is a saccharide with one monomeric unit (monosaccharide), and DP2 is a saccharide with two monomeric units (disaccharide). DP-n is expressed as a weight percent of an individual saccharide on a total carbohydrate (also referred to interchangeably herein as "saccharide") dry weight basis.

According to a second embodiment, the saccharide oligomers especially have a ratio of (DP3-5)/DP6+ less than 0.23.

According to a third embodiment, the saccharide oligomers especially have a Mw ranging from 2000 to 3000 daltons and an Mn ranging from 900 to 1500 daltons.

The term "Mw" refers to the weight average molecular weight.

The term "Mn" refers to the number average molecular weight, which is the average molecular weight of all components of the saccharide oligomers.

According to a fourth embodiment, the saccharide oligomers especially have a fiber content of between 60 and 90 %, more preferably 70 to 85 %.

Non-limitingly, the saccharide oligomers according to the invention, or able to be obtained according to the method defined below, may be incorporated into compositions or products intended for ingestion and for oral administration, such as various foodstuffs, for instance confectionery, pastries, ice creams, chewing pastes, chewing gums, drinks, jellies, soups, milk-based preparations, yoghurts, cakes, prepared animal fodder, dietary supplements, pharmaceutical, veterinary, dietary or hygiene products, such as, for example, elixirs, cough syrups, lozenges or tablets, pastilles, oral hygiene solutions, toothpastes and tooth gels.

A second subject of the invention is based on a process of preparing saccharide oligomers comprising the steps consisting of:
a) providing a saccharified corn starch syrup with a DP1 content of between 45 and 60 % by dry weight, a DP2 content of between 30 and 40 %,
b) optionally increasing the solids content of the aqueous solution resulting from step a) up to at least 70% by weight of the total weight thereof,
c) carrying out a heat treatment on the aqueous solution resulting from step b) at a temperature of between 100° C and 150° C with phosphoric acid in an acid ratio between 0.045 and 0.060 % by dry weight, under absolute pressure between 200 to 1000 mbar,
d) optionally decolorization and demineralization of the solution of step c),
e) carrying a fiber enrichment method chosen in the group consisting of chromatography, membrane filtration with or without preliminary enzymatic treatment,
f) optionally concentration and drying of the resulted solution.

The first step of the method according to the invention consists in providing a saccharified corn starch syrup with a DP1 content of between 45 and 60 % by dry weight, a DP2 content of between 30 and 40 %.

The conditions under which corn starch is hydrolyzed by liquefaction and then saccharified to achieve this carbohydrate spectrum are well known to those skilled in the art.

The second step of the method according to the invention is optional since it consists, where appropriate, in increasing the solids content of the aqueous solution resulting from step a) up to at least 70% by weight of the total weight thereof.

Those skilled in the art will know to adapt the parameters of residence time, temperature and pressure, especially as a function of the initial solids content thereof and of the solids content which they desire to finally obtain.

The third step of the method according to the invention consists in carrying out a heat treatment on the aqueous solution resulting from step b) at a temperature of between 100° C and 150° C with an acid ratio between 0.045 and 0.060 % by dry weight, under an absolute pressure between 200 to 1000 mbar.

The polymerization reaction occurs under these conditions.

This step is carried out (batch or continuous process) in a polymerization reactor fitted with heating devices and which makes it possible to work at reduced pressure. Such a reactor may especially consist of a curing oven or a vacuum furnace. Alternatively, the operation of adjusting the solids content and of polymerization is carried out in the same reactor, which advantageously has the abovementioned means and devices.

In the fourth step of the method according to the invention, the decolorization and demineralization is done on active charcoal and strong anionic and cationic resins, as exemplified below.

The fifth step of the method according to the invention consists in a purification method chosen in the group consisting of chromatography, membrane filtration, with or without preliminary enzymatic treatment.

The preliminary enzymatic treatment consists of using an amylase to hydrolyze the terminal α-1,4 and α-1,6 D-glucose residues from the non-reducing ends of the saccharide chains.

This can be achieved by any method known to the man skilled in the art, using an enzyme such as amyloglucosidase from *Aspergillus niger.*

The last step consists in optionally concentration and drying during of the resulted solution by any method well known to the man skilled in the art.

A final subject of the present invention relates to the use of the saccharide oligomers according to the invention, or able to be obtained according to the method defined above, in human food and animal feed.

The examples which follow make it possible to better understand the present invention, without however limiting the scope thereof.

### EXAMPLE

### ANALYTICAL METHODS

Throughout the present application, the Mw, Mn, and PDI were determined by high pressure size-exclusion chromatography (HPSEC).

This method permits to separate molecules in solution by their size, which correlates to their molecular weight and are compared to Pullulan standards with known molecular weight.

Pullulan standards (Grade P-5, P-10, P-20, P-50, P-100, P-200, P-400 and P-800) were purchased from Showa Denko K.K. (Japan); glycerin, glucose, maltose, maltotriose, maltotetraose, maltopentaose, maltohexose and maltopentaose were purchased from Sigma Aldrich. 0.5 % standard solutions were prepared by dissolving 50 mg of each standard and 50 mg of glycerin (output marker) in 10.0 mL of eluent solvent and then filtered through a 0.45 micron filter.

Samples of the carbohydrate compositions were first de-ashed with 1.0 g LEWATIT S4228 and PUROLITE C 150 MBH mixed resins and then diluted with Eluent solvent to about 2.5 %, 0.25 % of glycerine (output marker) was added, followed by filtration through a 0.45 micron filter.

100 µL of either the standard solutions or sample solutions were injected into a HPLC system equipped with a refractive index detector and 3 SHODEX SEC columns (OHpak SB-805 HQ, SB-803 HQ, SB-802 HQ connected in series). The chromatography was performed with following parameters:
- Injecting volume: 100 µL
- Flowrate: 0.5 mL/min
- Column temperature: 35°C
- Eluent: sodium nitrate 0.1M + sodium azide 0.02 % filtered on a 0.02 micron filter
- Eluting time: 80 min

The resulting chromatograms were analyzed offline using Empower software. The liberated mono-, di-, oligo- and polysaccharides size is determined according to the eluting time comparing to glycerin, glucose, maltose, maltotriose, maltotetraose, maltopentaose, maltohexose, maltopentaose and Pullulans standard eluting time.

Throughout the present application, the content of mono- and di-saccharides (i.e., DP1 and DP2) was determined using HPLC by the following method :
Sample was diluted with deionized water at a refractometric index of 10.0 at 20°C and demineralized on cationic/anionic resin with 1.0 g ion exchange resins (LEWATIT S4228 and PUROLITE C 150 MBH), sample was diluted with deionized water at a refractometric index of 5.0 at 20°C, and filtered through a 0.45 micron filter before injection into the HPLC for DP carbohydrate analysis.

The injected volume is 20 µL. DP separation was accomplished using two BioRad Aminex HPX-87N, 300 mm × 7.8 mm columns (BioRad) in series using water as the eluent at a flow rate of 0.50 mL/min at 85°C for 80 minutes.

Separated DP fractions were quantitated with a refractive index detector.

The percent area of each peak relative to total areas of all peaks was used to quantitate each saccharide peak, with no refractive index response factor, the refractive index response for all saccharides being expected to be very similar.

Throughout the present application, the content of DP3 - DP5 was determined using HPLC by the following method :
Sample was diluted with deionized water at a refractometric index of 10.0 at 20°C and demineralized on cationic/anionic resin with 1,0 g ion exchange resins (LEWATIT S4228 and PUROLITE C 150 MBH), sample was diluted with deionized water at a refractometric index of 5.0 at 20°C, and filtered through a 0.45 micron filter before injection into the HPLC for DP carbohydrate analysis. The injected volume is 20 µL. DP separation was accomplished using two BioRad Aminex HPX-42A, 300 mm × 7.8 mm columns (BioRad) in series using water as the eluent at a flow rate of 0.40 mL/min at 55°C for 80 minutes. Separated DP fractions were quantitated with a refractive index detector. The percent area of each peak relative to total areas of all peaks was used to quantitate each saccharide peak, with no refractive index response factor.

Throughout the present application, the contents of glycosidic bonds are determined by the Hakomori method.

The Hakomori method makes it possible to access the contents of total 1,4-, 1,6-, 1,2- and 1,3-glycosidic bonds.

The Hakomori method is that described in the publication of 1964, j. Biol. Chem., 55, 205.

Throughout the present application, the fiber content is measured according to AOAC method 2001-03.

### Products according to the Invention

In tests nos. A to C, 3 products were produced according to the method in accordance with the present invention.

A native corn starch is classically liquefied and saccharified to obtain the following profile.

**Table 1**

| | For the production of Batch A | For the production of Batch B | For the production of Batch C |
|---|---|---|---|
| DP1 content (% dry weight) | 43.1 | 46.3 | 53.2 |
| DP2 content (% dry weight) | 35.8 | 32.7 | 39.4 |
| DP ≥ 3 * (% dry weight) | 21.1 | 21.0 | 7.4 |

| | | | |
|---|---|---|---|
| Note (*): this DP value ≥ 3 is calculated as follows: 100% - %DP1 - %DP2. | | | |

This syrup is prepared at 70% Dry Substance.

Syrup is mixed with phosphoric acid solution and continuously feed in a reactor "3VTECH Continuous Film Dryer EC-2000" and heat to a final temperature under vacuum to be polymerized.

Polymerized product is diluted at 30 % dry substance to obtain a liquid product and decolorized using active powder with a ratio of 5% w/w DS at a temperature of 40°C. Liquid Product is filtered to remove active carbon and to obtain a liquid product at coloration <500 ICUMSA.

Liquid product is demineralized at a temperature of 45°C using a strong acid cation resin, a weak base anion and a mixed bed with a strong acid cation and strong base anion resins to obtain a liquid product with a resistivity >100 kΩ/cm.

Liquid product is concentrated at 40% Dry Substance and enriched in fiber by removal of DP1-2 compounds. Concentrated liquid product is continuously feed at a flowrate of 450 kg/h in a SMB chromatography (10 columns of 120 liters) to separate an extract stream enriched in DP1-2 and a raffinate stream enriched in fibers.

The raffinate stream in evaporated to 70% dry substance to obtain liquid enriched fibers products.

The conditions of the polycondensation, purification and fiber enrichment are presented in the following table.

**Table 2**

| | | Batch A | Batch B | Batch C |
|---|---|---|---|---|
| | Flowrate (kg/h) | 300 | 300 | 300 |
| Polycondensation reaction | Final Temperature (°C) | 150 | 141 | 143 |
| | Pressure (mbar) | 600 | 825 | 800 |
| | Phosphoric acid * (% dry weight) | 0.052 | 0.055 | 0.055 |
| | Fiber content (%DS) | 68 | 71 | 74 |
| Purification | Decolorization | Active powder charcoal ACTICARBONE^{®} 3SA | | |
| | Demineralization | Strong Acid Cation (DOWEX^{®} 88) & Weak Base Anion (DOWEX^{®} 66) | | |
| | | Mixed bed strong anion (AMBERLITE^{®} FPA98 OH) and strong cation (DOWEX^{®} 88 resins | | |
| Fiber Enrichment | Chromatography | SMB with resin AmberLite^{™} CR1360 in sodium form | | |
| | DS yield (%) | 70 | 72 | 70 |

| | | | | |
|---|---|---|---|---|
| *Note: (*) the phosphoric acid 75* % *(density 1.579 kg*/*L*) *ratio added to the saccharified syrup is calculated as followed:* *Acid ratio = (acid phosphoric 75* % *quantity* / *phosphoric acid density* × *phosphoric acid concentration 75%)* / *(syrup quantity* × *dry matter* of *the syrup)* | | | | |

For each of the 3 batches obtained, the Applicant determined:
- The total amount of DP3 to DP5,
- The ratio (DP3-5)/DP6+,
- The ratio of the total number of 1 ,6-glycosidic bonds to the total number of 1 ,4-glycosidic bonds,
- The ratio of the total number of 1 ,4-glycosidic bonds to the total number of 1 ,2-glycosidic bonds,
- The Mw and Mn
- The fiber content in %

The applicant also determined these same parameters for the following products:
- NUTRIOSE FB06 sold by ROQUETTE
- FIBERSOL-2 sold by ADM
- PROMITOR SCF85 & SCF85C sold by TATE & LYLE
- STA-LITE POLYDEXTROSE sold by TATE & LYLE,
- LOGIFIBER Corn Fiber Powder sold by SMINGREDIENTS
- RESISTANT DEXTRIN BLB sold by BAOLINGBAO
- RESISTANT DEXTRIN SB sold by SHANDONG BAILONG

**Table 3**

| | DP1-2 (%) | DP3-5 (%) | (DP3-5) / DP6+ | 1,6/1,4 ratio | 1,4/1,2 ratio | Mn (dalton) | Mw (dalton) | Fiber content (%) |
|---|---|---|---|---|---|---|---|---|
| Test A | 6.3 | 12 | 0.15 | 0.6 | 4.8 | 1180 | 2930 | 77 |
| Test B | 4.4 | 11 | 0.12 | 1.0 | 3.5 | 1310 | 2750 | 81 |
| Test C | 7.0 | 13 | 0.16 | 1.7 | 1.9 | 1080 | 2365 | 84 |
| NUTRIOSE FB06 | 0.5 | 4 | 0.04 | 0.5 | 5.9 | 2320 | 4270 | 84 |
| FIBERSOL-2 | 3.9 | 7 | 0.08 | 0.7 | 4.0 | 1315 | 2970 | 91 |
| PROMITOR SCF85 | 3.5 | 13 | 0.15 | 1.0 | 2.8 | 1235 | 2290 | 86 |
| PROMITOR SCF85C | 2.1 | 9 | 0.11 | 1.1 | 2.2 | 1450 | 2560 | 88 |
| STA-LITE | 14.5 | 16 | 0.25 | 2.2 | 1.1 | 710 | 1670 | 78 |
| LOGIFIBER | 4.5 | 17 | 0.22 | 1.6 | 2.6 | 1275 | 2330 | 85 |
| RESISTANT DEXTRIN BLB | 2.5 | 24 | 0.34 | 1.5 | 1.7 | 1140 | 1625 | 93 |
| RESISTANT DEXTRIN SB | 2.3 | 18 | 0.24 | 1.6 | 2.0 | 1300 | 2640 | 86 |

The products according to the invention have a very low content of available glucose, have the ideal compromise between a product that is relatively fiber-rich and therefore perfectly suited to healthy balanced diets, and a product with a very low glucose bioavailability with regard to the organism and therefore perfectly suited to diabetic patients or those following diets aiming to reduce sensitivity to this pathological condition.

### Comparison analysis with products not according to the Invention

For comparison purpose, the saccharides developed in the patent application WO2014145276A1, that may be considered as the closest prior art, were also presented in the following table.

As the analytical methods developed in this international patent application differ in certain respects from the analytical methods used by the applicant, the values presented below have been measured using those of the patent application.

**Table 4**

| | DP1-2 (%) | DP3-5 (%) | (DP3-5) / DP6+ | 1,6/1,4 ratio | 1,4/1,2 ratio | Mw (dalton) | Digestibility (%) |
|---|---|---|---|---|---|---|---|
| Test A | 8.0 | 12 | 0.15 | 0.7 | 3.3 | 2555 | 16 |
| Test B | 6.0 | 11 | 0.12 | 1.2 | 3.7 | 2640 | 15 |
| Test C | 8.5 | 13 | 0.15 | 1.5 | 1.8 | 2310 | 11 |
| PROMITOR 85 | 4.9 | 16 | 0.20 | 0.6 | 3.9 | 2208 | 10 |
| POLYDEXTROSE | 19.0 | 16 | 0.25 | 2.6 | 1.0 | 1693 | 15 |
| 2A CARGILL | 18.4 | 41 | 1.02 | 2.1 | 1.5 | 1243 | 28 |
| 2B CARGILL | 12.1 | 23 | 0.36 | 2.1 | 13 | 1522 | 27 |
| WO2014145276A1 | < 30 | > 20 | > 0.25 | 1 - 4 | < 2 | 300 - 3200 | 5-50% |

It is possible to observe, remarkably, that all the fiber-rich commercial products belonging to the prior art have:
- a higher content of DP3-5 and (DP3-DP5)/DP6+ ratio,
- an alpha 1.2, alpha 1.4 and alpha 1.6 profile quite different
- a lower molecular weight
for an equivalent content of fiber.

So, the saccharide oligomers according to the invention offers an alternative to such compounds.

Finally, the 3 products according to the invention have a very low content of available glucose, this is particularly surprising due to the fact that although these novel products are fiber-rich, they have a lower content of these fibers than the other products tested. Thus, only these products have the ideal compromise between a product that is relatively fiber-rich and therefore perfectly suited to healthy balanced diets, and a product with a very low glucose bioavailability with regard to the organism and therefore perfectly suited to diabetic patients or those following diets aiming to reduce sensitivity to this pathological condition.

## Claims

1. Saccharide oligomers comprising:
- A total amount of DP1 - DP2 ranging from 5 and 10 %, preferably from 4 to 8 %, and a total amount of DP 3 to DP 5 ranging from 9 and 19 %, preferably from 10 to 15 %,
- A ratio of 1,6-glycosidic/1,4-glycosidic linkages ranging from 0.5 to about 2 preferably from 0.6 to 1.8, and a ratio of 1,4-glycosidic/1,2-glycosidic linkages ranging from 1,5 to 6, preferably from 1.9 to 5.

2. The saccharide oligomers as claimed in claim 1, wherein said saccharide are digestion resistant or slowly digestible.

3. The saccharide oligomers as claimed in claim 1 having a ratio of (DP3-5)/DP6+ less than 0.23.

4. The saccharide oligomers as claimed in claim 1, having a Mw ranging from 2000 to 3000 daltons and an Mn ranging from 900 to 1500 daltons.

5. The saccharide oligomers of any preceding claims comprising a total soluble dietary fiber concentration, measured according to AOAC method 2001-03, of between 60 and 90 %, more preferably 70 to 85 % on a dry weight basis.

6. A process for preparing the saccharide oligomers of any preceding claims, comprising the steps consisting of:
a) providing a saccharified corn starch syrup with a DP1 content of between 45 and 60 % by dry weight, a DP2 content of between 30 and 40 %,
b) optionally increasing the solids content of the aqueous solution resulting from step a) up to at least 70% by weight of the total weight thereof,
c) carrying out a heat treatment on the aqueous solution resulting from step b) at a temperature of between 100° C and 150° C with phosphoric acid in an acid ratio between 0.045 and 0.060 % by dry weight, under absolute pressure between 200 to 1000 mbar,
d) optionally decolorization and demineralization of the solution of step c),
e) carrying a fiber enrichment method chosen in the group consisting of chromatography, membrane filtration with or without preliminary enzymatic treatment,
f) optionally concentration and drying of the resulted solution.

7. A food composition comprising the saccharide oligomers of any of the claims 1 to 5.

8. The food composition of claim 7, wherein the food composition is a human food.

9. The food composition of claim 7, wherein the food composition is an animal feed.
